# FASCICULE DE BREVET EUROPEEN

(11) **EP 1 364 936 B1**
(45) Date de publication et mention de la délivrance du brevet: **19.04.2006**
(21) Numéro de dépôt: 03291103.4
(22) Date de dépôt: 09.05.2003
(51) Int. Cl.: C07C 51/12, B01J 12/00

(54) **Procédé de carbonylation des alcools utilisant un catalyseur à base de rhodium ou d'iridium dans un liquide ionique non-aqueux, avec un recyclage efficacé du catalyseur**
Verfahren zur Carbonylierung von Alkoholen, mittels eines Katalysators auf Rhodium- oder Iridium-Basis in einem nichtwässrigen ionischen Lösungsmittel, mit effizienter Wiederverwendung des Katalysators
Process for the carbonylation of alcohols using a rhodium or iridium based catalyst in an ionic, non-aqueous liquid, with an efficient recycling of the catalyst

(30) Priorité: 23.05.2002 FR 0206317
(43) Date de publication de la demande: 26.11.2003
(73) Titulaire: Institut Français du Pétrole, 92852 Rueil-Malmaison Cedex (FR)
(72) Inventeur: Magna, Lionel, 69007 Lyon (FR); Olivier Bourbigou, Hlène, 92500 Rueil Malmaison (FR); Harry, Stéphane, 78360 Montesson (FR); Commereuc, Dominique, 92190 Meudon (FR)

(56) Documents cités:
- EP-A- 0 338 730
- EP-A- 0 976 711
- GB-A- 2 029 409
- US-A- 4 366 259
- US-B1- 6 211 405

## Description

La présente invention concerne un procédé de carbonylation des alcools au moyen d'un catalyseur à base de rhodium ou d'iridium en présence d'un halogénure utilisé comme promoteur, mis en oeuvre en milieu liquide ionique, avec un recyclage efficace du catalyseur. Le système homogène obtenu en fin de réaction est distillé. Le pied de distillation est constitué par le catalyseur solubilisé dans un liquide ionique non-aqueux. Ce liquide ionique comprend au moins un cation ammonium et/ou phosphonium quaternaire Q⁺ et au moins un anion A⁻. Le catalyseur comprend au moins un complexe du rhodium ou de l'iridium et le promoteur est constitué par au moins un halogénure.

La carbonylation des alcools et notamment du méthanol est une réaction de grande importance industrielle pour la fabrication d'acide acétique utilisé dans un grand nombre d'applications. Ce produit intervient directement dans la fabrication d'acétate de vinyle et d'anhydride acétique et peut être utilisé comme solvant de réaction dans la production d'acide téréphtalique.

De multiples références traitent de la production d'acide acétique par carbonylation du méthanol. On citera par exemple US 6 211 405, qui décrit la carbonylation des alcools avec un catalyseur du rhodium et d'iridium, GB 2029409, qui décrit la carbonylation des alcools avec un catalyseur du ruthénium et les travaux de la société BASF à la fin des années 60 *(Hydrocarbon Processing, Nov 1966, vol.45, n°11, p141 et Hydrocarbon Processing, Nov 1973, p. 92*) qui décrivent la réaction de carbonylation du méthanol dans des conditions très dures (60 MPa, 230 °C) en présence d'un complexe à base de cobalt promu par un dérivé iodé. Dans les années 70, la société Monsanto a commercialisé un procédé amélioré (Monsanto. (1973) US 3.769.329) qui fonctionne dans des conditions de température et de pression beaucoup plus douces (3 à 4 MPa, 180-220 °C) en présence d'un complexe à base de rhodium promu par de l'iodure de méthyle. Ce procédé se distingue par le rendement très élevé en acide acétique (99 %) lié à la présence d'une concentration en eau importante dans le mélange réactionnel. En 1980, la société Hoechst-Celanese améliora le procédé Monsanto en ajoutant un promoteur à base d'iodure de lithium ou d'iodure de sodium (Hoechst Celanese Corp. (1991) US 5.001.259) ce qui permet de réduire considérablement les concentrations en eau nécessaire dans le procédé Monsanto et donc limiter les problèmes de formation des sous produits par réduction de la réaction parasite de « Water gas shift ». Enfin en 1996, BP chemicals commercialise un procédé à base d'iridium (Cativa™) comparable au procédé Monsanto à basse concentration en eau (Chem. Br. 32 (1996) 7 et Chem. Ind. (London) 483 (1996)). Ce procédé apporte des améliorations considérables par rapport aux précédentes technologies comme l'augmentation des vitesses de réaction, la réduction des sous produits liquides et l'augmentation du rendement par rapport au monoxyde de carbone.

La plupart de ces procédés ont recours à des catalyseurs homogènes dissous dans une phase organique constituée des réactifs et des produits ce qui rend parfois complexe et coûteux leurs séparations.

II a maintenant été trouvé que, dans la réaction de carbonylation des alcools catalysée par des complexes du rhodium ou de l'iridium, la mise en oeuvre dans un liquide ionique non-aqueux comprenant au moins un cation ammonium et/ou phosphonium quaternaire Q⁺ et au moins un anion A⁻, liquide à une température inférieure à 90 °C, permet un recyclage du métal dans le liquide ionique grandement amélioré. Le mélange homogène de fin de réaction peut être directement distillé. La faible tension de vapeur du liquide ionique et sa grande stabilité thermique permettent de récupérer en pied de colonne de distillation le catalyseur stabilisé et maintenu soluble dans le liquide ionique. A la fin de cette étape de distillation, la phase liquide ionique contenant le catalyseur peut être réutilisée.

Plus précisément, la présente invention a donc pour objet de fournir un procédé pour la carbonylation en phase liquide des alcools par le monoxyde de carbone défini par le fait qu'il comprend :
- la réaction de carbonylation effectuée dans une zone de réaction à une température de 50 à 150 °C sous une pression comprise entre 0,5 MPa et 20 MPa en présence d'au moins un catalyseur comprenant au moins un complexe du rhodium et/ou de l'iridium et un agent promoteur halogéné dans au moins un liquide ionique non-aqueux comprenant au moins un sel de formule générale Q⁺A⁻, dans laquelle Q⁺ représente un ammonium quaternaire et/ou un phosphonium quaternaire, ledit sel ayant un point de fusion inférieur à 90 °C ;
- la séparation du liquide ionique non-aqueux contenant au moins la majeure partie du catalyseur ; et
- le recyclage du liquide ionique non-aqueux contenant au moins la majeure partie du catalyseur ainsi séparé vers la zone de réaction.

Sans vouloir être lié par une quelconque théorie, on peut penser que dans les conditions de la réaction de carbonylation, sous pression de monoxyde de carbone, le catalyseur est présent en grande partie sous forme du complexe [Rh(CO)₂I₂]⁻ (si le précurseur est à base de rhodium) ou de [Ir(CO)₂I₂]⁻ (si le précurseur est à base d'iridium) qui, compte tenu de son caractère ionique, reste très soluble et stable dans la phase sel fondu pendant et après distillation, et ce, malgré l'absence de tout ligand additionnel, tel qu'une phosphine ou un phosphite, destiné à augmenter la stabilité du complexe pendant et après distillation.

Le liquide ionique non-aqueux est choisi dans le groupe formé par les sels liquides qui ont pour formule générale Q⁺ A⁻ dans laquelle Q⁺ représente un ammonium quaternaire et/ou un phosphonium quaternaire et A⁻ représente tout anion susceptible de former un sel liquide à basse température, c'est-à-dire en dessous de 90 °C et avantageusement d'au plus 85 °C, et de préférence en dessous de 50 °C. Les anions A⁻ préférés sont les ions nitrate, sulfate, phosphate, acétate, halogénoacétates, tétrafluoroborate, tétrachoroborate, tétraarylborate, tétraalkylborate, hexafluorophosphate, hexafluoroantimonate, fluorosulfonate, alkylsulfonates, perfluoroalkylsulfonates, bis(perfluoroalkylsulfonyl)-amidures et arènesulfonates, ces derniers éventuellement substitués par des groupements halogénés ou halogénoalkyles. Avec la même condition que le sel soit liquide à au-dessous de 90 °C, l'anion A⁻ peut également être un halogénure, par exemple un iodure. Dans ce cas, il importe que le liquide ionique joue le rôle de solvant et représente plus de 20 % en poids du système catalytique.

Les ammoniums et/ou phosphoniums quaternaires Q⁺ répondent de préférence aux formules générales NR¹R²R³R⁴⁺ et PR¹R²R³R⁴⁺, ou aux formules générales R¹ R²N=CR³R⁴⁺ et R¹ R²P=CR³R⁴⁺ dans lesquelles R¹,R², R³ et R⁴, identiques ou différents, représentent l'hydrogène (à l'exception du cation NH₄⁺ pour NR¹ R²R³R⁴⁺), de préférence un seul substituant représentant l'hydrogène, ou des restes hydrocarbyles ayant de 1 à 30 atomes de carbone, par exemple des groupements alkyles, saturés ou non saturés, cycloalkyles ou aromatiques, aryle ou aralkyle, éventuellement substitués, comprenant de 1 à 30 atomes de carbone. Les ammonium et/ou phosphonium peuvent également être dérivés d'hétérocycles azotés et/ou phosphorés comportant 1, 2 ou 3 atomes d'azote et/ou de phosphore, dans lesquelles les cycles sont constitués de 4 à 10 atomes, de préférence 5 à 6 atomes.

L'ammonium ou le phosphonium quaternaire peuvent également être un cation répondant à l'une des formules générales :

R¹R²⁺N=CR³-R⁵-R³C=N+R¹R²

et

R¹R²+P=CR³-R⁵-R³C=P+R¹R²

dans lesquelles R¹, R² et R³, identiques ou différents, sont définis comme précédemment et R⁵ représente un reste alkylène ou phénylène. Parmi les groupements R¹, R², R³ et R⁴ on mentionnera les radicaux méthyle, éthyle, propyle, isopropyle, butyle, secondaire butyle, tertiaire butyle, amyle, méthylène, éthylidène, phényle ou benzyle; R⁵ pourra être un groupement méthylène, éthylène, propylène ou phénylène.

Le cation ammonium et/ou phosphonium Q⁺ est choisi de préférence dans le groupe formé par le N-butylpyridinium, le N-éthylpyridinium, le pyridinium, l'éthyl-3'-méthyl-1-imidazollum, le butyl-3-méthyl-1-imidazolium, l'hexyl-3-méthyl-1-imidazolium, le butyl-3-diméthyl-1,2-imidazolium, le diéthyl-pyrazolium, le N-butyl-N-méthylpyrrolidinium, le triméthylphényl-ammonium, le tétrabutylphosphonium, le tributyl-tétradécyl-phosphonium.

A titre d'exemples des sels utilisables selon l'invention on peut citer l'hexafluorophosphate de N-butyl-pyridinium, le tétrafluoroborate de N-éthyl-pyridinium, le fluorosulfonate de pyridinium, le tétrafluoroborate de butyl-3-méthyl-1-imidazolium, l'hexafluoro-antimonate de butyl-3-méthyl-1-imidazolium, l'hexafluorophosphate de butyl-3-méthyl-1-imidazolium, le trifluoroacétate de butyl-3-méthyl-1-imidazolium, le trifluorométhylsulfonate de butyl-3-méthyl-1-imidazolium, le bis(trifluorométhylsulfonyl)amidure de butyl-3-méthyl-1-imidazolium, l'hexafluorophosphate de triméthyl-phénylammonium et le tétrafluoroborate de tétrabutylphosphonium. Ces sels peuvent être utilisés seuls ou en mélange.

N'importe quelle source de rhodium ou d'iridium qui réagira avec le monoxyde de carbone dans le milieu réactionnel pour donner un complexe rhodium-carbonyle ou iridium carbonyle peut être utilisé dans le cadre de la présente invention. Les composés du rhodium et de l'iridium précurseurs du catalyseur sont par exemple choisis dans le groupe formé par leurs sels comme les halogénures, acétylacétonates, les carboxylates et en particulier le formiate ou l'acétate, les complexes carbonyle comme l'acétylacétonate de rhodium dicarbonyle, et les clusters carbonyles. Le choix du composé métallique précurseur n'est pas critique, mais on préfère en général utiliser les halogénures.

Tous les halogènes ou les composés halogénés utilisés comme agent promoteur du précurseur catalytique sont susceptibles de convenir au système décrit selon la présente invention. Toutefois, on préfère ceux qui contiennent de l'iode et du brome. Les agents promoteurs possibles sont ceux qui répondent à la formule RXₙ où n est compris entre 1 et 3, R est un groupement alkyle ou aromatique et X un atome de chlore, de brome ou d'iode (par exemple CH₃I, C₆H₅Br, CH₃CH₂I, ICH₂CH₂I, etc). On pourra également utiliser les agents promoteurs de formules X₂ ou X₃ pour lesquels X est un atome d'iode, de chlore ou de brome (par exemple Br₂, I₂, I₃ etc). Les acides correspondants de formule HX font également partie des agents promoteurs utilisables pour la présente invention (par exemple HI, HBr ou HCI).

La composition catalytique est obtenue par mélange, d'une manière quelconque, du liquide ionique avec le composé du rhodium et/ou de l'iridium et l'halogénure (agent promoteur). On peut également dissoudre préalablement le composé du métal de transition dans un solvant organique. Ce solvant organique peut être ajouté en large excès et utilisé comme solvant de réaction.

La vitesse de la réaction dépend de la concentration en catalyseur dans le système. La concentration en rhodium et/ou en iridium est avantageusement comprise entre 0,1 mmole par litre et 5 moles par litre, de préférence entre 1 mmole et 1 mole par litre, et encore entre 10 et 500 mmoles par litre. Le rapport molaire entre le promoteur halogéné et le composé du rhodium est compris entre 0,1:1 et 1000:1, de préférence entre 1:1 et 500:1.

Les matières premières utilisées de préférence pour la réaction de carbonylation, selon la présente invention, sont, par exemple des alcools aliphatiques ayant de 1 à 20 atomes de carbone et des alcools aromatiques ayant de 6 à 20 atomes de carbone, tels que le méthanol, l'éthanol, le propanol, l'isopropanol, les butanols, les pentanols, l'alcool benzylique, le phényl-1 éthanol et ses dérivés, le phénol et les hexanols, également des alcools supérieurs, tels que les décanols, et leurs formes isomères. Cependant, un alcool constitue la matière première privilégiée. Par exemple, si on désire obtenir de l'acide acétique ou ses dérivés, la substance de départ peut être de l'alcool méthylique ou ses dérivés, tels que l'éther diméthylique, l'acétate de méthyle, l'iodure de méthyle et/ou des combinaisons de ces substances.

Selon la présente invention, la réaction de carbonylation est effectuée de préférence sous une pression de monoxyde de carbone entre 1 et 10 MPa.

La réaction catalytique de carbonylation des alcools peut être conduite en système fermé, en système semi-ouvert ou en continu avec un ou plusieurs étages de réaction. Dans une mise en oeuvre en continu, l'effluent du réacteur sous pression est transféré dans une enceinte de dépressurisation où il est dépressurisé jusqu'à une pression qui peut être aussi faible que 0,5 MPa, à une température au plus égale à 100 °C et de préférence inférieure à 60 °C. Le contenu de ce dépressuriseur est ensuite distillé.

A la sortie de la zone de distillation, la phase liquide ionique non-aqueux contenant la presque totalité du catalyseur est, au moins en partie, retournée au réacteur, l'autre partie pouvant être traitée pour éliminer des résidus de décomposition du catalyseur en très faible quantité et d'éventuels sous-produits lourds.

L'invention a également pour objet une installation pour la mise en oeuvre du procédé de carbonylation tel que défini dans la description qui précède, ladite installation comportant :
- au moins un réacteur A1 ;
- au moins une enceinte de dépressurisation (« dépressuriseur ») B1 ;
- et au moins une enceinte de distillation A2 pour la séparation des produits de réaction et du liquide ionique contenant au moins le catalyseur qui est recyclé au réacteur A1 ;
ainsi que :
- au moins une conduite 1 pour l'introduction de la charge à carbonyler et du monoxyde de carbone;
- au moins une conduite 2 pour le transfert de l'effluent du réacteur vers le dépressuriseur B1 ;
- au moins une conduite 3 pour envoyer vers l'enceinte de distillation A2 le mélange de l'effluent organique et du solvant ionique contenu dans le dépressuriseur B1 ;
- au moins une conduite 6 pour renvoyer vers le réacteur A1 les gaz issus du dépressuriseur B1 ;
- au moins une conduite 5 permettant de renvoyer dans le réacteur A1 le pied de flash contenant au moins le liquide ionique et le catalyseur séparée dans A2.

L'installation comprend en outre :
- dans la section de séparation, au moins une colonne A2 pour la séparation des produits bruts de la réaction de l'alcool à carbonyler qui n'a pas réagi, de l'agent promoteur ; ainsi que :

- au moins une conduite 4 pour recycler vers le réacteur A1 l'alcool à carbonyler et le promoteur qui n'ont pas réagi séparés dans la colonne A2 ;
- au moins une conduite 7 permettant d'envoyer les produits sortant en tête de la colonne A2 dans la suite du train de fractionnement des produits.

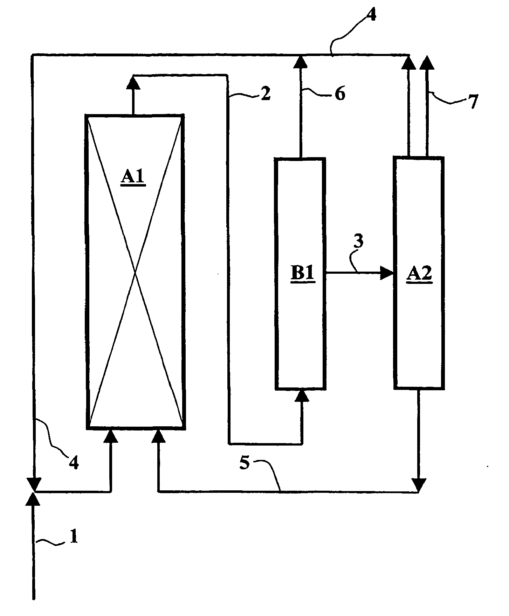

On comprendra mieux le procédé et l'installation de l'invention à partir de la description qui en est faite ci-après, en liaison avec la Figure 1. Selon la Figure 1, la réaction est réalisée dans le réacteur A1 en présence de la charge à carbonyler, qui peut être introduite par la ligne 1, du (ou des) composé(s) de métal de transition, de monoxyde de carbone, qui peuvent être introduits par la ligne 1, et en présence d'au moins un liquide ionique non-aqueux ainsi que de l'agent promoteur halogéné. Le liquide ionique peut être introduit dans le réacteur au début de la réaction. Optionnellement, du liquide ionique frais peut être injecté dans le réacteur A1 au cours de la réaction et du liquide ionique usé soutiré de A1 (les moyens d'injection et de soutirage du liquide ionique ne sont pas indiqués dans la Figure 1).

La chaleur de réaction est éliminée par les techniques connues de l'homme du métier qui ne sont pas représentées sur la Figure 1.

A la sortie de la section réactionnelle, l'effluent du réacteur est envoyé, par la ligne 2, dans au moins un dépressurisseur B1 dans lequel la pression est abaissée. Une agitation peut être maintenue dans B1, soit mécaniquement, soit par tout autre moyen convenable. Les gaz libérés par la dépressurisation s'échappent par la ligne 6 et sont renvoyés vers le réacteur A1 après avoir été recomprimés.

L'effluent du dépressurieur B1 est envoyé dans la colonne de distillation A2 par la ligne 3. Dans cette colonne A2, on sépare en tête l'alcool à carbonyler qui n'a pas réagi ainsi qu'une partie de l'agent promoteur halogéné et les produits de carbonylation. L'alcool ainsi que l'agent promoteur halogéné peuvent être recyclés au réacteur A1 par la ligne 4. Les produits bruts de la réaction recueillis en A2 sont envoyés dans un train de fractionnement spécifique (non représenté) par la ligne 7.

En pied de colonne A2, la phase polaire qui contient au moins le liquide ionique et le catalyseur est récupérée et renvoyée au réacteur A1 par la ligne 5.

Les exemples suivants illustrent l'invention sans en limiter la portée.

### EXEMPLE 1

La réaction de carbonylation est conduite dans un autoclave en acier inoxydable recouvert d'un revêtement vitrifié d'une contenance de 100 mL, muni d'une double enveloppe permettant la régulation de la température par circulation d'un fluide caloporteur, et équipé d'une agitation magnétique efficace. Dans cet autoclave purgé au préalable de l'air et de l'humidité et placé sous pression atmosphérique du monoxyde carbone, on introduit une solution contenant 200 mg de RhCl₃.3H₂O (soit 0,76 mmole de rhodium), 5 mL d'iodométhane, 5 mL de bis(trifluorométhylsulfonyl) amidure de butyl-3-méthyl-1-imidazolium [BMI][Tf₂N] et 13 mL de méthanol. On porte la pression de monoxyde de carbone à 3 MPa et la température à 130 °C et on met l'agitation en route. Après 2 heures de réaction, on ferme l'arrivée de monoxyde de carbone et on refroidit rapidement le réacteur jusqu'à 25 °C. Après soutirage hors de l'autoclave, l'effluent est homogène monophasique de couleur rouge.

La totalité du système est introduite dans un montage de distillation flash. Le distillat se présente sous la forme d'un liquide homogène pratiquement incolore et le pied de flash sous la forme d'un liquide limpide homogène rouge vif. Cette coloration dénote la présence de l'ion (Rh (CO)₂I₂)⁻ solubilisé dans le liquide ionique. L'analyse par chromatographie en phase gazeuse du distillat et la pesée des résidus non flashés (liquide ionique + rhodium) permettent d'établir le bilan matière de la réaction. La conversion du méthanol est de 99,6 % en poids. La sélectivité est de 96,1 % en acide acétique et 3,9 % en acétate de méthyle.

### EXEMPLE 2 (comparatif)

La réaction d'hydroformylation est conduite dans le même appareillage et selon le même mode opératoire que décrit dans l'Exemple 1, à ceci près qu'il a été réalisé en absence de liquide ionique. Après soutirage hors de l'autoclave, l'effluent est homogène monophasique de couleur rouge.

La totalité du système est introduite dans un montage de distillation flash. Le distillat se présente sous la forme d'un liquide homogène rouge et le pied de flash sous la forme d'un solide noir comprenant du rhodium métallique solide. L'analyse par chromatographie en phase gazeuse du distillat et la pesée des résidus non flashés (rhodium) permettent d'établir le bilan matière de la réaction. La conversion du méthanol est de 99,6 % en poids. La sélectivité est de 93,6 % en acide acétique et de 6,4 % en acétate de méthyle.

### EXEMPLE 3

La réaction de carbonylation du méthanol est conduite dans le même appareillage et selon le même mode opératoire que décrit dans l'Exemple 1, à ceci près que l'on rajoute 10 mL d'eau distillée dans le système et que la durée de réaction est portée à 3 h. Après soutirage hors de l'autoclave, l'effluent est homogène monophasique de couleur rouge.

La totalité du système est introduite dans un montage de distillation flash. Le distillat se présente sous la forme d'un liquide homogène pratiquement incolore et le pied de flash sous la forme d'un liquide limpide homogène rouge vif. L'analyse par chromatographie en phase gazeuse du distillat et la pesée des résidus non flashés (liquide ionique + rhodium) permettent d'établir le bilan matière de la réaction. La conversion du méthanol est de 99,5 % en poids. La sélectivité est de 99,5 % en acétaldéhyde et de 0,5 % en acétate de méthyle.

### EXEMPLE 4 : Recyclage

La réaction de carbonylation du méthanol est conduite dans le même appareillage et selon le même mode opératoire que décrit dans l'Exemple 1. La phase liquide ionique contenant le catalyseur récupérée en pied de colonne de distillation, est recyclée vers le réacteur pour un nouveau cycle. On rajoute alors 5 mL d'iodométhane et 13 mL de méthanol frais. Aucun apport de liquide ionique n'est nécessaire.

Trois cycles consécutifs ont été réalisés. Ils sont résumés dans le tableau suivant :

| Cycle | Liquide ionique | Temp (°C) | P (MPa) | Temps (h) | Conversion (%) | Sélectivité (%) | |
|---|---|---|---|---|---|---|---|
| | | | | | | CH₃CO₂H | CH₃CO₂CH₃ |
| 1 | [BMI][TF₂N] | 130 | 3 | 2h | 99,6 | 96 | 4 |
| 2 | [BMI][TF₂N] | 130 | 3 | 2h | 99,5 | 96 | 4 |
| 3 | [BMI][TF₂N] | 130 | 3 | 2h | 99,7 | 97 | 3 |

## Revendications

1. Procédé pour la carbonylation en phase liquide des alcools par le monoxyde de carbone comprennant:
- la réaction de carbonylation effectuée dans une zone de réaction à une température de 50 à 150°C sous une pression comprise entre 0,5 MPa et 20 MPa en présence d'au moins un système catalytique comprenant au moins un complexe du rhodium et/ou de l'iridium et un agent promoteur halogéné dans au moins un liquide ionique non-aqueux comprenant au moins un sel de formule générale Q⁺A⁻, dans laquelle Q⁺ représente un ammonium quaternaire et/ou un phosphonium quaternaire, ledit sel ayant un point de fusion inférieur à 90 °C
- la séparation du liquide ionique non-aqueux contenant au moins la majeure partie du catalyseur ; et
- le recyclage du liquide ionique non-aqueux contenant au moins la majeure partie du catalyseur ainsi séparé vers la zone de réaction.

2. Procédé selon la revendication 1 **caractérisé en ce que**, dans la formule du liquide ionique non-aqueux Q⁺A⁻, l'anion A- est choisi parmi les ions halogénures, nitrate, sulfate, phosphate, acétate, halogénoacétates, tétrafluoroborate, tétrachoroborate, hexafluorophosphate, hexafluoroantimonate, fluorosulfonate, alkylsulfonates, perfluoroalkylsulfonates, bis(perfluoroalkylsulfonyl)amidures, arènesulfonates, et arènesulfonates substitués par des groupements halogènes ou halogénoalkyles.

3. Procédé selon la revendication 2 **caractérisé en ce que** l'anion A⁻ est l'anion iodure et le liquide ionique représente plus de 20 % en poids du système catalytique.

4. Procédé selon la revendication 1 à 3 **caractérisé en ce que** le cation ammonium et/ou phosphonium quaternaire répond à l'une des formules générales :
NR¹R²R³R⁴⁺ et PR¹ R²R³R⁴⁺,
et
R¹R²N=CR³R⁴⁺ et R¹R²P=CR³R⁴⁺
dans lesquelles R¹, R², R³ et R⁴, identiques ou différents, représentent chacun l'atome d'hydrogène (le cation NH₄⁺ étant exclu et de préférence un seul substituant représentant l'atome d'hydrogène) ou un reste hydrocarbyle ayant de 1 à 30 atomes de carbone.

5. Procédé selon la revendication 4 **caractérisé en ce que** le cation ammonium et/ou phosphonium est dérivé d'un hétérocycle azoté et/ou phosphoré comportant 1, 2 ou 3 atomes d'azote et/ou de phosphore, dans lequel le (ou les) cycle(s) est (sont) constitué(s) de 4 à 10 atomes.

6. Procédé selon l'une des revendications 1 à 3 **caractérisé en ce que** le cation ammonium et/ou phosphonium quaternaire répond à l'une des formules générales :
R¹R²+N=CR³-R⁵-R³C=N+R¹R²
et
R¹R²+P=CR³-R⁵-R³C=P+R¹R²
dans lesquelles R¹, R² et R³, identiques ou différents, représentent l'atome d'hydrogène ou un reste hydrocarbyle ayant de 1 à 30 atomes de carbone et R⁵ représente un reste alkylène ou phénylène.

7. Procédé selon l'une des revendications 1 à 3 **caractérisé en ce que** le cation ammonium et/ou phosphonium est choisi parmi le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le diéthyl-pyrazolium, le N-butyl-N-méthylpyrrolidinium, le triméthylphényl-ammonium, le tétrabutyl-phosphonium et le tributyl-tétradécyl-phosphonium.

8. Procédé selon l'une des revendications 1 à 3 **caractérisé en ce que** le liquide ionique non-aqueux est choisi parmi l'hexafluorophosphate de N-butyl-pyridinium, le tétrafluoroborate de N-éthyl-pyridinium, le fluorosulfonate de pyridinium, le tétrafluoroborate de butyl-3-méthyl-1-imidazolium, l'hexafluoroantimonate de butyl-3-méthyl-1-imidazolium, l'hexafluorophosphate de butyl-3-méthyl-1-imidazolium, le trifluoroacétate de butyl-3-méthyl-1-imidazolium, le trifluorométhylsulfonate de butyl-3-méthyl-1-imidazolium, le bis(trifluorométhylsulfonyl)amidure de butyl-3-méthyl-1-imidazolium, l'hexafluorophosphate de triméthylphénylammonium et le tétrafluoroborate de tétrabutylphosphonium.

9. Procédé selon l'une des revendications 1 à 8 **caractérisé en ce que** le composé du rhodium ou de l'iridium précurseur du catalyseur est choisi le parmi les halogénures, les acétylacétonates, les carboxylates, leurs complexes carbonyles et leurs clusters carbonyles.

10. Procédé selon l'une des revendications 1 à 9 **caractérisé en ce que** la concentration du complexe précurseur du catalyseur dans le liquide ionique est comprise entre 0,1 mmole par litre et 5 moles par litre.

11. Procédé selon l'une des revendications 1 à 10 **caractérisé en ce que** le composé halogéné utilisé comme agent promoteur du précurseur catalytique est choisi parmi ceux qui répondent à la formule RXn où n est compris entre 1 et 3, R est un groupement alkyle ou aromatique et X un atome de chlore, de brome ou d'iode, ceux qui répondent aux formules X2 ou X3 pour lesquelles X est un atome d'iode, de chlore ou de brome et les acides correspondants de formule HX.

12. Procédé selon l'une des revendications 1 à 11 **caractérisé en ce que** le composé à carbonyler est choisi parmi les alcools aliphatiques ayant de 1 à 20 atomes de carbone et des alcools aromatiques ayant de 6 à 20 atomes de carbone.

13. Procédé selon la revendication 12 **caractérisé en ce que** l'alcool est choisi parmi le méthanol, l'éthanol, le propanol, l'isopropanol, les butanols, les pentanols, l'alcool benzylique, le phényl-1 éthanol et ses dérivés, le phénol et les hexanols, les alcools supérieurs et leurs formes isomères.

14. Procédé selon l'une des revendications 1 à 13 **caractérisé en ce que** le liquide ionique non-aqueux contenant la presque totalité du catalyseur est séparé par distillation.

15. Procédé selon l'une des revendications 1 à 14 **caractérisé en ce que** le liquide ionique non-aqueux contenant la presque totalité du catalyseur séparé est recyclé vers la réaction de carbonylation.

16. Installation pour la mise en oeuvre d'un procédé de carbonylation selon l'une des revendications 1 à 15 **caractérisée en ce qu'**elle comprend :
- au moins un réacteur (A1);
- au moins une enceinte de dépressurisation (« dépressuriseur ») (B1);
- et au moins une enceinte de distillation (A2) pour la séparation des produits de réaction et du liquide ionique contenant au moins le catalyseur qui est recyclé au réacteur (A1);
ainsi que :
- au moins une conduite (1) pour l'introduction de la charge à carbonyler et du monoxyde de carbone;
- au moins une conduite (2) pour le transfert de l'effluent du réacteur vers le dépressuriseur (B1);
- au moins une conduite (3) pour envoyer vers l'enceinte de distillation (A2) le mélange de l'effluent organique et du solvant ionique contenu dans le dépressuriseur (B1);
- au moins une conduite (6) pour renvoyer vers le réacteur (A1) les gaz issus du dépressuriseur (B1);
- au moins une conduite (5) permettant de renvoyer dans le réacteur(A1) le pied de flash contenant au moins le liquide ionique et le catalyseur séparé dans (A2).

17. Installation selon la revendication 16 **caractérisée en ce qu'**elle comprend en outre :
- dans la section de séparation, au moins une colonne (A2) pour la séparation des produits bruts de la réaction de l'alcool à carbonyler qui n'a pas réagi, de l'agent promoteur ;
ainsi que :
- au moins une conduite (4) pour recycler vers le réacteur (A1) l'alcool à carbonyler et le promoteur qui n'ont pas réagi séparés dans la colonne (A2);
- au moins une conduite (7) permettant d'envoyer les produits sortant en tête de la colonne (A2) dans la suite du train de fractionnement des produits.

## Patentansprüche

1. Verfahren zur Carbonylierung in flüssiger Phase von Alkoholen durch ein Kohlenmonoxid, umfassend:
- die Carbonylierungsreaktion, die in einer Reaktionszone bei einer Temperatur zwischen 50 und 150 °C unter einem Druck zwischen 0,5 MPa.und 20 MPa in Gegenwart mindestens eines katalytischen Systems durchgeführt wird, das mindestens einen Rhodium- und/oder Iridiumkomplex und einen halogenierten Promoter in mindestens einer nicht-wässrigen ionischen Flüssigkeit umfasst, der mindestens ein Salz mit der allgemeinen Formel Q⁺A⁻ umfasst, wobei Q⁺ ein quartäres Ammonium und/oder ein quartäres Phosphonium darstellt, wobei das Salz einen Schmelzpunkt unter 90 °C aufweist,
- die Separation der nicht-wässrigen ionischen Flüssigkeit, die mindestens den größten Teil des Katalysators enthält, und
- die Rezyklierung der nicht-wässrigen ionischen Flüssigkeit, die mindestens den größten Teil des Katalysators enthält, der so zur Reaktionszone hin separiert wird.

2. Verfahren nach Anspruch 1, **dadurch gekennzeichnet, dass** in der Formel der nicht-wässrigen ionischen Flüssigkeit Q⁺A⁻ das Anion A⁻ ausgewählt wird aus Halogenidionen, Nitraten, Sulfaten, Phosphaten, Acetaten, Halogenacetaten, Tetrafluorboraten, Tetrachloroboraten, Hexafluorphosphaten, Hexafluorantimonaten, Fluorsulfonaten, Alkylsulfonaten, Perfluoroalkylsulfonaten, bis(Perfluoralkylsulfonyl)amiden, Arenesulfonaten und Arenesulfonaten, die durch Halogen- oder Halogenalkylgruppen substituiert werden.

3. Verfahren nach Anspruch 2, **dadurch gekennzeichnet, dass** das Anion A⁻ ein Jodidanion ist, und die ionische Flüssigkeit mehr als 20 Gew.-% des katalytischen Systems darstellt.

4. Verfahren nach Anspruch 1 bis 3, **dadurch gekennzeichnet, dass** das Ammoniumkation und/oder das quartäre Phosphonium einer der folgenden allgemeinen Formeln entspricht:
NR¹R²R³R⁴⁺ und PR¹R²R³R⁴⁺,
und
R¹R²N=CR³R⁴⁺ und R¹R²P=CR³R⁴⁺
worin R¹, R², R³ und R⁴, gleich oder verschieden, jeweils das Wasserstoffatom darstellen (wobei das Kation NH₄⁺ ausgeschlossen ist und vorzugsweise ein einziger Substituent das Wasserstoffatom darstellt) oder ein Hydrocarbylrest, der 1 bis 30 Kohlenstoffatome aufweist.

5. Verfahren nach Anspruch 4, **dadurch gekennzeichnet, dass** das Ammonium- und/oder Phosphoniumkation von einer heterocyclischen Stickstoff- und/oder Phosphorverbindung abgeleitet ist, das 1, 2 oder 3 Stickstoff- und/oder Phosphoratome umfasst, in dem der Zyklus (oder die Zyklen) aus 4 bis 10 Atomen besteht (bestehen).

6. Verfahren nach einem der Anspruche 1 bis 3, **dadurch gekennzeichnet, dass** das Ammoniumkation und/oder das quartäre Phosphonium einer der folgenden allgemeinen Formeln entspricht:
R¹R²+N=CR³-R⁵-R³C-N⁺R¹R²
und
R¹R²⁺P=CR³-R⁵-R³C=P⁺R¹R²
worin R¹, R² und R³, gleich oder verschieden, das Wasserstoffatom oder einen Hydrocarbylrest darstellen, der 1 bis 30 Kohlenstoffatome aufweist, und R⁵ einen Alkyl- oder Phenylenrest darstellt.

7. Verfahren nach einem der Ansprüche 1 bis 3, **dadurch gekennzeichnet, dass** das Ammonium- und/oder Phosphoniumkation ausgewählt wird aus N-Butylpyridinium, N-Ethylpyridinium, Pyridinium, Ethyl-3-methyl-1-imidazolium, Butyl-3-methyl-1-imidazolium, Hexyl-3-methyl-1-imidazolium, Butyl-3-dimethyl-1,2-imidazolium, Diethylpyrazolium, N-Butyl-N-methylpyrrolidinium, Trimethylphenylammonium, Tetrabutylphosphonium und Tributyltetradecylphosphonium.

8. Verfahren nach einem der Ansprüche 1 bis 3, **dadurch gekennzeichnet, dass** die nicht-wässrige ionische Flüssigkeit ausgewählt wird aus Hexafluorphosphat von N-Butylpyridinium, Tetrafluorborat von N-Ethylpyridinium, Fluorsulfonat von Pyridinium, Tetrafluorborat von Butyl-3-methyl-1-imidazolium, Hexafluorantimonat von Butyl-3-methyl-1-imidazolium, Hexafluorphosphat von Butyl-3-methyl-1-imidazolium, Trifluoracetat von Butyl-3-methyl-1-imidazolium, Trifluormethylsulfonat von Butyl-3-methyl-1-imidazolium, bis(Trifluormethylsulfonyl)amid von Butyl-3-methyl-1-imidazolium, Hexafluorphosphat von Trimethylphenylammonium und Tetrafluorborat von Tetrabutylphosphonium.

9. Verfahren nach einem der Ansprüche 1 bis 8, **dadurch gekennzeichnet, dass** die Rhodium- oder Iridiumverbindung, Vorläufersubstanz des Katalysators, ausgewählt wird aus Halogeniden, Acetylacetonaten, Carboxylaten, ihren Carbonylkomplexen und ihren Carbonylclustern.

10. Verfahren nach einem der Ansprüche 1 bis 9, **dadurch gekennzeichnet, dass** die Konzentration des Vorläuferkomplexes des Katalysators in der ionischen Flüssigkeit zwischen 0,1 mMol pro Liter und 5 Mol pro Liter liegt.

11. Verfahren nach einem der Ansprüche 1 bis 10, **dadurch gekennzeichnet, dass** die als Promoter des katalytischen Vorläufers verwendete Halogenverbindung ausgewählt wird aus jenen Verbindungen, die der Formel RXn entsprechen, worin n zwischen 1 und 3 liegt, R eine Alkylgruppe oder eine aromatische Gruppe und X ein Chlor-, Brom- oder Jodatom ist, die den Formeln X2 oder X3 entsprechen, worin X ein Jod-, Chlor- oder Bromatom ist und die Säuren der entsprechenden Formel HX.

12. Verfahren nach einem der Ansprüche 1 bis 11, **dadurch gekennzeichnet, dass** die zu carbonylierende Verbindung ausgewählt wird aus den aliphatischen Alkoholen, die zwischen 1 und 20 Kohlenstoffatome aufweisen und aromatischen Alkoholen, die zwischen 6 und 20 Kohlenstoffatome aufweisen.

13. Verfahren nach Anspruch 12, **dadurch gekennzeichnet, dass** der Alkohol ausgewählt wird aus Methanol, Ethanol, Propanol, Isopropanol, Butanolen, Pentanolen, Benzylalkohol, Phenyl-1-ethanol und dessen Derivaten, Phenol und Hexanolen, höheren Alkoholen und deren isomeren Formen.

14. Verfahren nach einem der Ansprüche 1 bis 13, **dadurch gekennzeichnet, dass** die nicht-wässrige ionische Flüssigkeit, die fast die gesamte Katalysatormenge enthält, durch Destillation getrennt wird.

15. Verfahren nach einem der Ansprüche 1 bis 14, **dadurch gekennzeichnet, dass** die nicht-wässrige ionische Flüssigkeit, die fast die gesamte Menge des Katalysators enthält, zur Carbonylisierungsreaktion hin rezykliert wird.

16. Vorrichtung zur Durchführung des Verfahrens zur Carbonylisierung nach einem der Ansprüche 1 bis 15, **dadurch gekennzeichnet, dass** sie folgendes umfasst:
- mindestens einen Reaktor A1;
- mindestens einen Druckabbaubehälter ("Druckabbauer") (B1);
- und mindestens einen Destillationsbehälter (A2) für die Separation der Reaktionsprodukte von der ionischen Flüssigkeit, die mindestens den Katalysator enthält, der im Reaktor (A1) rezykliert wird;
sowie:
- mindestens eine Leitung (1) für die Einführung der zu carbonylierenden Ladung und des Kohlenmonoxids;
- mindestens eine Leitung (2) für den Transport der Abwässer des Reaktors in den Druckabbauer (B1);
- mindestens eine Leitung (3), um die Mischung des organischen Abwassers und des ionischen Lösemittels, das im Druckabbauer (B1) enthalten ist, zum Destillationsbehälter (A2) zu leiten;
- mindestens eine Leitung (6), um die aus dem Druckabbauer (B1) austretenden Gase zum Reaktor (A1) zu leiten;
- mindestens eine Leitung (5), die es ermöglicht, den Flash-Fuß in den Reaktor (A1) zurückzuleiten, der mindestens die ionische Flüssigkeit enthält und den in (A2) getrennten Katalysator.

17. Vorrichtung nach Anspruch 16, **dadurch gekennzeichnet, dass** sie außerdem umfasst:
- im Separationsabschnitt, mindestens eine Säule (A2) für die Separation der Rohprodukte der Reaktion des zu carbonylierenden Alkohols, der nicht reagiert hat, vom Promoter;
sowie:
- mindestens eine Leitung (4), um den zu carbonylierenden Alkohol und den Promoter, die nicht reagiert haben, separiert in der Säule (A2), zum Reaktor (A1) hin zu rezyklieren;
- mindestens eine Leitung (7), die es ermöglicht, die Produkte, die an der Spitze der Säule (A2) austreten, danach wieder in den Fraktionierungsablauf der Produkte zu leiten.

## Claims

1. A process for carbonylating alcohols with carbon monoxide in the liquid phase, in comprising:
• carrying out the carbonylation reaction in a reaction zone at a temperature of 50°C to 150°C at a pressure in the range 0.5 MPa to 20 MPa in the presence of at least one catalyst comprising at least one rhodium and/or iridium complex and a halogenated promoter in at least one non-aqueous ionic liquid comprising at least one salt with general formula Q⁺A⁻, in which Q⁺ represents a quaternary ammonium and/or a quaternary phosphonium cation, said salt having a melting point of less than 90°C;
• separating the non-aqueous ionic liquid containing at least the major portion of the catalyst; and
• recycling the separated non-aqueous ionic liquid containing at least the major portion of the catalyst to the reaction zone.

2. A process according to claim 1, **characterized in that**, in the formula for the non-aqueous ionic liquid Q⁺ A⁻, the anion A⁻ is selected from halides, nitrate, sulphate, phosphate, acetate, halogenoacetates, tetrafluoroborate, tetrachloroborate, hexafluorophosphate, hexafluoroantimonate, fluorosulphonate, alkylsulphonates, perfluoroalkylsulphonates, bis(perfluoroalkylsulphonyl)amides and arenesulphonates and arenesulphonates substituted with halogen or halogenoalkyl groups.

3. A process according to claim 2, **characterized in that** the anion A⁻ is iodide anion and the ionic liquid constitutes more than 20% by weight of the catalyst system.

4. A process according to one of claims 1 to 3, **characterized in that** the quaternary ammonium and/or phosphonium cation has one of the following general formulae:
NR¹R²R³R⁴⁺ and PR¹R²R³R⁴⁺,
and
R¹R²N = CR³R⁴⁺ and R¹R²P = CR³R⁴⁺
in which R¹, R², R³ and R⁴, which may be identical or different, each represent a hydrogen atom (the cation NH₄⁺ being excluded, and preferably a single substituent representing a hydrogen atom) or a hydrocarbyl residue containing 1 to 30 carbon atoms.

5. A process according to claim 4, **characterized in that** the ammonium and/or phosphonium cation is derived from a nitrogen-containing and/or phosphorus-containing heterocycle containing 1, 2 or 3 nitrogen and/or phosphorus atoms, in which the cycle or cycles is/are constituted by 4 to 10 atoms.

6. A process according to one of claims 1 to 3, **characterized in that** the quaternary ammonium and/or phosphonium cation has one of the following general formulae:
R¹R²⁺N = CR³-R⁵-R³C = N⁺R¹R²
v
R¹R²⁺P = CR³-R⁵-R³C = P⁺R¹R²
in which R¹, R² and R³, which may be identical or different, represent a hydrogen atom or a hydrocarbyl residue containing 1 to 30 carbon atoms, and R⁵ represents an alkylene or phenylene residue.

7. A process according to one of claims 1 to 3, **characterized in that** the ammonium and/or phosphonium cation is selected from N-butylpyridinium, N-ethylpyridinium, pyridinium, 3-ethyl-1-methylimidazolium, 3-butyl-1-methylimidazolium, 3-hexyl-1-methylimidazolium, 3-butyl-1,2-dimethylimidazolium, diethylpyrazolium, N-butyl-N-methylpyrrolidinium, trimethylphenylammonium, tetrabutylphosphonium and tributyl-tetradecylphosphonium.

8. A process according to one of claims 1 to 3, **characterized in that** the non-aqueous ionic liquid is selected from N-butylpyridinium hexafluorophosphate, N-ethyl pyridinium tetrafluoroborate, pyridinium fluorosulfonate, 3-butyl-1-methylimidazolium tetrafluoroborate, 3-butyl-1-methylimidazolium hexafluoroantimonate, 3-butyl-1-methylimidazolium hexafluorophosphate, 3-butyl-1-methylimidazolium trifluoroacetate, 3-butyl-1-methylimidazolium trifluoromethylsulphonate, 3-butyl-1-methylimidazolium bis(trifluoromethylsulfonyl)amide, trimethylphenylammonium hexafluorophosphate and tetrabutylphosphonium tetrafluoroborate.

9. A process according to one of claims 1 to 8, **characterized in that** the rhodium or iridium catalyst precursor compound is selected from halides, acetylacetonates, carboxylates, their carbonyl complexes, and carbonyl clusters.

10. A process according to one of claims 1 to 9, **characterized in that** the concentration of catalyst precursor complex in the ionic liquid is in the range 0.1 mmoles per litre to 5 moles per litre.

11. A process according to one of claims 1 to 10, **characterized in that** the halogenated compound used as the promoter for the catalyst precursor is selected from those with formula RXₙ in which n is in the range 1 to 3, R is an alkyl or aromatic group and X is a chlorine, bromine or iodine atom, those with formulae X2 or X3 in which X is an iodine, chlorine or bromine atom, and the corresponding acids with formula HX.

12. A process according to one of claims 1 to 11, **characterized in that** the compound to be carbonylated is selected from aliphatic alcohols containing 1 to 20 carbon atoms and aromatic alcohols containing 6 to 20 carbon atoms.

13. A process according to claim 12, **characterized in that** the alcohol is selected from methanol, ethanol, propanol, isopropanol, butanols, pentanols, benzyl alcohol, 1-phenyl ethanol and its derivatives, phenol and hexanols, higher alcohols, and their isomeric forms.

14. A process according to one of claims 1 to 13, **characterized in that** the non-aqueous ionic liquid containing almost all of the catalyst is separated by distillation.

15. An process according to one of claims 1 to 14, **characterized in that** the non-aqueous ionic liquid containing almost all of the separated catalyst is recycled to the carbonylation reaction.

16. An apparatus for carrying out the carbonylation process according to one of claims 1 to 15, **characterized in that** it comprises:
• at least one reactor (A1);
• at least one depressurization vessel ("depressurizer") (B1);
• and at least one distillation chamber (A2) to separate the reaction products from the ionic liquid containing at least the catalyst, which is recycled to reactor (A1);
and also:
• at least one line (1) for introducing feed to be carbonylated and carbon monoxide;
• at least one line (2) for transferring the effluent from the reactor to the depressurizer (B1);
• at least one line (3) for sending the mixture of the organic effluent and the ionic solvent contained in the depressurizer (B1) to the distillation chamber (A2);
• at least one line (6) for returning the gas from the depressurizer (B1) to the reactor (A1);
• at least one line (5) for returning the flash residue containing at least the ionic liquid and the catalyst separated in (A2) to the reactor (A1).

17. An apparatus according to claim 16, **characterized in that** it further comprises:
• in the separation section, at least one column (A2) for separating the crude reaction products from the unreacted alcohol to be carbonylated and the promoter agent;
and also:
• at least one line (4) for recycling the unreacted alcohol to be carbonylated and promoter separated in column (A2) to the reactor (A1);
• at least one line (7) for sending the products leaving from the head of the column (A2) to the remainder of the product fractionation train.
